# EUROPEAN PATENT SPECIFICATION

(11) **EP 3 164 118 B1**
(45) Date of publication and mention of the grant of the patent: **29.12.2021**
(21) Application number: 15736023.1
(22) Date of filing: 01.07.2015
(51) Int. Cl.: A61K 31/485, A61K 9/50

(54) **COATED PARTICLES**
BESCHICHTETE PARTIKEL
PARTICULES ENROBÉES

(30) Priority: 01.07.2014 GB 201411704
(43) Date of publication of application: 10.05.2017
(73) Proprietor: Lucideon Limited, Penkhull, Stoke on Trent ST4 7LQ (GB)
(72) Inventor: JACKSON, Philip Robert, Penkhull Stoke on Trent ST4 7LQ (GB); CRESSWELL, Mark, Penkhull Stoke on Trent ST4 7LQ (GB); CAMPBELL, Ian F., Penkhull Stoke on Trent ST4 7LQ (GB)
(74) Representative: HGF
(86) International application number: PCT/GB2015/051930
(87) International publication number: WO 2016/001670

(56) References cited:
- EP-A1- 1 674 094
- WO-A1-2009/036812
- WO-A1-2013/001536
- US-A- 5 851 670
- US-A1- 2012 135 077
- None

## Description

### INTRODUCTION

The present invention relates to porous particles comprising a coating formed from a glassy material, and their methods of manufacture. More particularly, the invention relates to coated porous particles comprising an active ingredient, wherein the coating exhibits tuneable solubility characteristics in aqueous and alcoholic media.

### BACKGROUND OF THE INVENTION

The abuse or misuse of medications represents an ongoing challenge for public health authorities. Whether intentional or accidental, the improper use of prescription medicaments has the potential to cause serious harm, ranging from reduced efficacy of the drug, to an increased expression of side effects and addictions.

Drug abusers have devised a variety of ways for achieving the "high" associated with improper substance use. A primitive, yet effective, technique sees a user crush or pulverize one or more oral dosages for subsequent administration via other routes, such as snorting, smoking or injecting. More elaborate methods involve extracting active ingredients from pharmaceuticals with the aid of household solvents, and even kitchen appliances, such as microwaves.

The threat to public health posed by improper drug use has prompted numerous public health authorities to task drug manufacturers with developing improved tamper-proof technologies. One approach has been to provide analgesic compositions comprising both agonistic and antagonistic ingredients, with the antagonistic effect designed to dominate when the composition is administered by an abusive route, such as by injection.

Other tamper-proof techniques have focused around so-called aversion technologies, which aim to discourage the would-be abuser by making the process more difficult and less pleasurable. Such approaches have involved using gelling agents to prevent a user from drawing the substance into a syringe, or including additives to cause increased burning and irritation in the nasal passages when snorted.

However, with abuse rates having quadrupled in the decade from 1990 to 2000 ^{1,2}, there remains a constant need for improved tamper-resistant technologies.

The present invention was devised with the foregoing in mind.

### SUMMARY OF THE INVENTION

According to a first aspect of the present invention there is provided a particle comprising:
a core substrate having a plurality of pores, and
a coating substantially encapsulating the core substrate,
the core substrate comprising at least one active ingredient present within the pores, and the coating being formed from an alkali phosphate glass or an alkali silicate glass, wherein the core substrate is selected from the group consisting of amorphous silica, glasses, clays, zeolites and ceramics and wherein the alkali phosphate or alkali silicate has a greater dissolution rate in aqueous media than in alcoholic media.

According to a second aspect of the present invention there is provided a process for the preparation of a plurality of particles as claimed in any preceding claim, the process comprising the steps of:
a) providing a plurality of core substrates each comprising a plurality of pores as claimed in any preceding claims, wherein the pores comprise at least one active ingredient as claimed in any preceding claim, and
b) contacting the plurality of core substrates with an alkali phosphate glass or an alkali silicate glass as claimed in any preceding claim, such that the plurality of core substrates become coated with the alkali phosphate glass or the alkali silicate glass.

According to a third aspect of the present invention there is provided tamper-proof, or abuse-deterrent, particles comprising coated particles as defined herein, the coated particles comprising at least one active ingredient.

According to a fourth aspect of the present invention there is provided a solid dosage form comprising a particle defined herein.

### DETAILED DESCRIPTION OF THE INVENTION

### Particles of the invention

As described hereinbefore, the present invention provides a particle comprising:
a core substrate having a plurality of pores, and
a coating substantially encapsulating the core substrate,
the core substrate comprising at least one active ingredient present within the pores, and the coating being formed from an alkali phosphate glass or an alkali silicate glass, wherein the core substrate is selected from the group consisting of amorphous silica, glasses, clays, zeolites and ceramics and wherein the alkali phosphate or alkali silicate has a greater dissolution rate in aqueous media than in alcoholic media.

In the particles of the present invention, the dissolution rate of the alkali phosphate glass or alkali silicate glass in aqueous media is greater than it is in alcoholic media. The dissolution rate of the alkali phosphate glass or alkali silicate glass in aqueous and alcoholic media can be determined by techniques well known in the art. In an embodiment, the dissolution rate may be determined by immersing a given quantity of the alkali phosphate glass or alkali silicate glass sample in a given quantity of an aqueous or alcoholic media for a given period of time (e.g. 5, 10, 20 or 30 minutes) and then determining the dissolution rate by either:
(i) analysing a given quantity of the aqueous media and alcoholic media (e.g. by ICP-OES analysis) to detect the quantity of dissolved alkali phosphate or alkali silicate in the media at the given time; and/or
(ii) by collecting, drying and weighing the amount of undissolved glass in a given volume of the suspension at a given point in time (e.g. 5, 10, 20 or 30 minutes) to determine the mass of undissolved glass that remains.

In an embodiment, a suspension (e.g. a 1% w/v suspension) of the alkali phosphate or alkali silicate glass (optionally with a particle size within the range of 30 to 250 microns) is dispersed in a given quantity of an aqueous media (e.g. phosphate buffered saline) or an alcoholic media (e.g. 40% ethanol in 0.1M HCI) for a given period of time (e.g. 5, 10, 20 or 30 minutes), and the amount of dissolution is determined by either:
(i) analysing a given quantity of the aqueous media and alcoholic media (e.g. by ICP-OES analysis) to detect the quantity of dissolved alkali phosphate or alkali silicate in the media at the given time; and/or
(ii) by collecting, drying and weighing the amount of undissolved glass in a given volume of the suspension at a given point in time (e.g. 5, 10, 20 or 30 minutes) to determine the mass of undissolved glass that remains.
A specific protocol for determining the dissolution rate is provided in Example 2 herein.

It will be understood that the phrase "substantially encapsulating the core substrate" relates to either or both of the scenarios where (i) the coating covers a substantial part of the outermost surface of the core substrate, and (ii) the coating blocks, partially blocks or impregnates all or a substantial number of pores present in the core substrate in a manner which restricts the availability of the active ingredient(s) contained therein to the alcoholic medium.

The solubility characteristics of particles of the invention present a number of advantages, most notably in the field of drug delivery. Until now, drug abusers have been endowed with a variety of methods for extracting active ingredients from prescription pharmaceuticals, which may then be concentrated to higher dosages for subsequent recreational use. Perhaps the most effective technique involves the use of one or more solvents to leach out the active ingredients from high-dosage controlled-release prescription medicaments. This so-called "dose-dumping" may also occur accidentally, whereby the simultaneous consumption of particular solvents, often ethanol present in alcoholic beverages, can induce the medicament to release its load almost instantaneously. Whether intentional or accidental, dose-dumping of this type can lead to abnormal quantities of the active ingredient in the blood stream, provoking a loss of efficacy, or an increased risk of side-effects and dependencies. WO2009/036812 discloses a pH-dependent controlled release composition comprising an opioid-containing core and a polymeric coating layer which is resistant to ethanol. The coating comprises a mixture of a water-insoluble vinyl polymer, a water-soluble anionic polymer and a non-porous inert lubricant.

The present invention now provides a novel means of significantly reducing the viability of such dose-dumping techniques by using particles comprising a core having a plurality of pores (which serves to retard the release of the drug) and a coating of particular glass having tuneable solubility characteristics in both alcoholic and aqueous media. When compared with the drug release profile under physiological conditions, the poor dissolution rate of the glass coating in alcohol, coupled with the slow release of drug from the porous core if/when the coating or portions of the coating has dissolved, serve to make such dose-dumping techniques impractical, if not impossible. The particles of the invention thereby present a means of realising tamper-proof and abuse-deterrent medicaments. Furthermore, by varying the porosity of the core substrate as well as the quantity of coating material, the particles of the invention allow the release profile of the active ingredient(s) to be tailored according to a patient's needs.

Suitably, the alkali phosphate glass and alkali silicate glass comprise at least one oxide selected from alkali metal oxides and alkaline earth metal oxides. In one embodiment, the alkali phosphate and alkali silicate glasses comprise only one oxide. Suitably, the oxide is Na₂O. Such binary systems balance ease of manufacture with the ability to offer a range of solubility characteristics. In another embodiment, the alkali phosphate and alkali silicate glasses comprise Na₂O, and optionally one or more other oxides selected from suitable glass-modifying oxides such as calcium oxide and magnesium oxide. Such ternary and quaternary systems offer a greater degree of flexibility depending on the desired solubility characteristics of the coating.

In another embodiment, the alkali silicate glass has a weight ratio of silica to total alkali oxide of 9:1 to 1:1. Suitably, the alkali silicate glass has a weight ratio of silica to total alkali oxide of 4:1 to 1:1. More suitably, the alkali silicate glass has a weight ratio of silica to total alkali oxide of 4:1 to 1.25:1. Within these weight ratios, the solubility of the material can be engineered to offer the desired coating characteristics.

In another embodiment, the alkali phosphate glass comprises 30-50 mol% phosphate. In another embodiment, the alkali phosphate glass is a metaphosphate or a pyrophosphate. Suitably, the alkali phosphate glass comprises 40-48 mol% phosphate. Suitably, the phosphate is provided as phosphorus pentoxide (P₂O₅).

In another embodiment, the alkali phosphate glass comprises 50-70 mol% oxide selected from alkali metal oxides and alkaline earth metal oxides. Suitably, the alkali phosphate glass comprises 52-60 mol% oxide selected from alkali metal oxides and alkaline earth metal oxides. More suitably, the alkali phosphate glass comprises 54 mol% oxide selected from alkali metal oxides and alkaline earth metal oxides. Suitably, the oxide is Na₂O.

In another embodiment, the coating is formed from an alkali phosphate glass consisting of 46 mol% P₂O5 and 54 mol% Na₂O.

In another embodiment, the at least one active ingredient is a pharmaceutically active compound. Suitably, the active ingredient is present in a quantity designed for extended release dosage, which may be an opioid or a non-opioid (e.g. pseudoephedrine). More suitably, the active ingredient is an opioid, opium derivative, or an opiate drug, including their isomers, esters, ethers and any salts thereof. More suitably, the at least one active ingredient is a drug defined as a "controlled substance" in the USA Controlled Substance Act, the Single Convention on Narcotic Drugs 1961 or the Misuse of Drugs Act 1971. Even more suitably, the active ingredient comprises one or more of oxycodone, hydrocodone, oxymorphone, morphine, or codeine, or a pharmaceutically acceptable salt thereof. Most suitably, the active ingredient is oxycodone, or a pharmaceutically acceptable salt thereof. Ethanol dose-dumping is an issue for almost all extended release medicaments, whose high dosages present an attractive target for drug misusers. Although opioid painkillers, in particular oxycodone, are incredibly potent analgesics, they can also lead to devastating addictions, thereby underlining the importance of the tamper-proof particles disclosed herein.

In another embodiment, the at least one active ingredient may be a non-opioid drug, such as a stimulant (e.g. pseudoephedrine).

In another embodiment, in addition to the at least one active ingredient, the particle comprises one or more other compounds, such as, for example, pharmaceutically-acceptable excipients.

In another embodiment, the core substrate may comprise two or more active ingredients, and optionally one or more other compounds, such as, for example, pharmaceutically-acceptable excipients.

The core substrate is selected from the group consisting of amorphous silica, glasses, clays, zeolites and ceramics. As described herein, the core substrate is selected from a group of inorganic materials that could be naturally occurring or synthesized via (i) ambient / moderate temperature or (ii) high temperature processing. Examples of (i) include zeolites or cylindrical clay structures such as hectorites, foamed ceramics and porous ceramics generated via sol-gel processing (the last of these either with or without sacrificial organics that are subsequently sintered out). Examples of (ii) include soluble glasses, and glasses that have undergone phase separation and chemical treatment to remove the lower durability phase. As described herein, the core substrate is an inorganic material offering either or both of (a) dissolution or (b) diffusion from a porous substrate. In an embodiment, the core substrate is an amorphous silica. More suitably, the core substrate is created via a tetraalkylorthosilicate monomer or a trialkylorthosilicate containing a single "organic" entity on the fourth bond to silicon. Most suitably, the core substrate is created via a tetraethyl orthosilicate monomer.

In another embodiment, the particle is a microparticle. Smaller particles offer enhanced tamper-proof characteristics by virtue of their reduced crushability. Suitably, the particle has a diameter of 50 - 350 µm. More suitably, the particle has a diameter of 125 - 250 µm.

In another embodiment, the particle comprises 1 - 10 wt% of the coating. Suitably, wherein the particle comprises 1 - 5 wt% of the coating. Most suitably, the particle comprises 2 - 5 wt% of the coating.

In another embodiment, the plurality of pores each have a diameter of 1.5 - 50 nm. Suitably, the plurality of pores each have a diameter of 1.5 - 30 nm.

In another embodiment, the core substrate has a pore volume of 1 x 10⁻³ - 10 cm³g⁻¹. Suitably, the core substrate has a pore volume of 5 x 10⁻³ - 5 cm³g⁻¹.

As described herein, the present invention also provides a product (e.g. a particle) obtainable, obtained, or directly obtained, by a process defined herein.

### Processes of the invention

As described hereinbefore, the present invention provides a process for the preparation of a plurality of particles as defined herein, the process comprising the steps of:
a) providing a plurality of core substrates each comprising a plurality of pores as claimed in any preceding claims, wherein the pores comprise at least one active ingredient as defined herein, and
b) contacting the plurality of core substrates with an alkali phosphate glass or an alkali silicate glass as defined herein, such that the plurality of core substrates become coated with the alkali phosphate glass or the alkali silicate glass.

In one embodiment, step b) comprises the step of fluidizing the plurality of core substrates in the presence of an alkali phosphate glass or an alkali silicate glass as claimed in any preceding claim, such that the plurality of core substrates become coated with the alkali phosphate glass or the alkali silicate glass. Suitably, the plurality of core substrates are fluidized in the presence of a sprayed solution of an alkali phosphate glass or an alkali silicate glass. In one embodiment, the solution of alkali phosphate or alkali silicate is sprayed onto the core substrates from above. In an alternative embodiment, the solution of alkali phosphate or alkali silicate is sprayed onto the core substrates from a column (e.g. a Würster column) provided within the fluidizing chamber.

The core substrates forming part of the invention can be coated via a variety of methods, some of which yield better results than others. Perhaps the crudest coating method involves mixing the plurality of core substrates with a solution of the glass coating materials and then evaporating away the solvent. Although still a viable technique, the aforementioned process is hampered by the need to mill the resulting material back to the desired particle size, thereby running the risk of creating freshly cleaved uncoated surfaces, which may have an adverse effect on the release profile of the active ingredient(s). The coating process can be improved by employing a technique which coats the core substrates individually. The fluidisation embodiment of the present invention involves fluidising the plurality of core substrates (keeping particles in dynamic motions and so apart from each other) and then spraying a solution of the glass coating material either on top of, or preferably within, the fluidised bed. Atomised droplets of glass solution land on individual core substrates and are instantly evaporated. The continual fluidisation and movement of the core substrates ensures no agglomeration.

In an embodiment, step a) comprises forming a plurality of core substrates each comprising a plurality of pores as defined herein, wherein the pores comprise at least one active ingredient as defined herein, and wherein the at least one active ingredient is introduced into the pores of the core substrate either during the formation of the core substrate or after the formation of the core substrate.

Suitably, the core substrates provided in step a) are prepared according to a sol-gel protocol.

In another embodiment, step a) comprises the steps of:
a1) providing a liquid mixture of core substrate precursors,
a2) subjecting the liquid mixture to conditions suitable to form a plurality of core substrates, and
a3) isolating the resulting core substrates comprising the at least one active ingredient,
wherein the at least one active ingredient is introduced either before, during or after step a2).

Suitably, step a1) comprises providing a mixture of core substrate precursors in an aqueous solution having a pH of 0-11.

Suitably, the at least one active ingredient is contacted with the liquid mixture of core substrate precursors prior to step a2). More suitably, the at least one active ingredient is in aqueous solution.

In one embodiment, the core substrate precursors are either identical or different. Suitably, the core substrate precursors are tetraethylorthosilicate (TEOS) monomers.

In another embodiment, step a2) comprises contacting the liquid mixture with the at least one active ingredient and stirring the resulting biphasic mixture. Suitably, the solution is stirred at room temperature. More suitably, the solution is stirred for 48 - 96 hours. Most suitably, the solution is stirred until a gel is formed.

In another embodiment, step a3) comprises drying the resulting core substrates, then milling the dried core substrates. Suitably, the core substrates resulting from step a2) are dried at a temperature of 30 - 90°C for 20 - 100 hours. More suitably, the core substrates resulting from step a2) are dried at a temperature of 50 - 70°C for 12 - 36 hours. Suitably, the dried core substrates are milled, and optionally sieved, to a particle size of 50 - 350 µm. More suitably, the dried core substrates are milled, and optionally sieved, to a particle size of 125 - 250 µm. Optionally, step a3) comprises one or more additional drying steps either during or after milling. Suitably, the one or more additional drying steps comprises heating the core substrates at a temperature of 30 - 100°C for 12 - 72 hours.

In another embodiment, the alkali phosphate glass or alkali silicate glass used in step b) is provided as an aqueous solution comprising 3-25% (m/v) of the glass.

In another embodiment, step b) comprises introducing the solution of alkali phosphate glass or alkali silicate glass to a fluidized bed of core substrates. Suitably, the solution of alkali phosphate glass or alkali silicate glass is introduced under sufficient pressure to provide a mist, or droplets, of glass. More suitably, the solution of alkali phosphate glass or alkali silicate glass is introduced to the fluidized bed of core substrates at a pressure of 0.8-1.5 bar. Most suitably, the solution of alkali phosphate glass or alkali silicate glass is introduced to the fluidized bed of core substrates at a pressure of 1.1-1.3 bar.

In another embodiment, the exhaust air and plurality of core substrates are maintained at a temperature of 30 - 50°C during fluidization. Suitably, the exhaust air and plurality of core substrates are maintained at a temperature of 35 - 47°C during fluidization.

In another embodiment, the core substrates are maintained at a temperature of 30 - 50°C during fluidization. Suitably, the core substrates are maintained at a temperature of 35 - 47°C during fluidization.

In another embodiment, the aqueous solution of glass is sprayed into the fluidization chamber using a peristaltic pump at a rate of 2 - 5 rpm.

### Applications

As described hereinbefore, the present invention also provides tamper-proof, or abuse-deterrent, particles comprising coated particles as defined herein, the coated particles comprising at least one active ingredient.

In an embodiment, the at least one active ingredient is a drug.

The tamper-proof or abuse-deterrent particles of the present invention present an effective means of reducing, or even eliminating, the viability of dose-dumping drug misuse techniques. The coated particles prevent a user from achieving a rapid extraction of an active ingredient, either *in vitro* (i.e. intentionally), or *in vivo* (i.e. accidentally), thereby reducing the risk of users developing health issues linked to side effects, dependencies, or reduced efficacy of drugs. The solubility characteristics of the particle's coating in aqueous media mean that the efficacy of the solid dosage under physiological conditions is not compromised.

In another embodiment, the tamper-proof/abuse-deterrent particles are provided as a solid dosage form.

As described hereinbefore, the present invention also provides a solid dosage form comprising a particle defined herein, wherein the particle comprises at least one pharmaceutically active compound.

In one embodiment, the solid dosage form is intended for oral or sublingual administration.

In another embodiment, the solid dosage form comprises a particle defined herein, wherein the particle comprises two or more pharmaceutically active compounds.

In another embodiment, the solid dosage form is an extended release dosage. Given that extended release dosage often contain high quantities of active ingredients, they pose an attractive target for drug misusers.

In another embodiment, the solid dosage form comprises at least one opioid active ingredient. Although opioids are widely used for their analgesic benefits, they are increasingly targeting by drug misusers. Suitably, the opioid is oxycodone or a pharmaceutically acceptable salt thereof.

In another embodiment, the solid dosage form is suitable for use as, or alongside, an abuse-deterrent medicaments.

### EXAMPLES

Examples of the invention will now be described, for the purpose of reference and illustration only, with reference to the accompanying figures, in which:
Fig. 1 illustrates sodium phosphate mass loss as a function of time in pH 6.8 phosphate buffer for sodium phosphate glass powder as prepared in Example 2 herein. Three repeat runs were carried out (as depicted by lines 1, 2 and 3) and an average mass loss was determined.
Fig. 2 illustrates sodium phosphate mass loss as a function of time in 40% EtOH/0.1 M HCI for sodium phosphate glass powder as prepared in Example 2 herein. Two repeat runs were carried out (as depicted by lines 1 and 2) and an average mass loss was determined.
Fig. 3 illustrates potassium silicate (2.05:1) mass loss as a function of time in pH 6.8 phosphate buffer and 40% EtOH/0.1 M HCI for potassium silicate glass powder as prepared in Example 2 herein.
Fig. 4 illustrates the percentage dissolution of four 1% (w/v) glass suspensions comprising a potassium silicate (1.43:1) in both pH 6.8 phosphate buffer and 40% EtOH/0.1 M HCI. The glass suspensions were made up and stirred for 5, 10, 20 and 30 minutes before being filtered and the collected solids dried in the oven at 80-95°C overnight. Each test was carried out independently and 3 replicates were carried out for each individual time point.
Fig. 5 illustrates oxycodone release from uncoated particles as a function of time in pH 6.8 phosphate buffer for core particle formulations 1 to 11 as defined herein.
Fig. 6 illustrates oxycodone release from uncoated particles as a function of time in 40% EtOH/0.1 M HCI for core particle formulations 1 to 11 as defined herein.
Fig. 7 illustrates oxycodone release from coated core particle formulations 1 to 11 as a function of time in pH 6.8 phosphate buffer. Particle formulations 1 to 11 shown have varying levels of sodium phosphate glass coating, as detailed in Tables 1 and 5 herein.
Fig. 8 illustrates oxycodone release from coated core particle formulations 1 to 11 as a function of time in 40% EtOH/0.1 M HCI. Particle formulations 1 to 11 shown have varying levels of sodium phosphate glass coating, as detailed in Tables 1 and 5 herein.
Fig. 9 compares oxycodone release from coated particle formulation 1 as a function of time in pH 6.8 phosphate buffer and in 40% EtOH/0.1 M HCl.
Fig. 10 compares oxycodone release from coated particle formulation 2 as a function of time in pH 6.8 phosphate buffer and in 40% EtOH/0.1 M HCI.
Fig. 11 compares oxycodone release from coated particle formulation 3 as a function of time in pH 6.8 phosphate buffer and in 40% EtOH/0.1 M HCI.
Fig. 12 compares oxycodone release from coated particle formulation 4 as a function of time in pH 6.8 phosphate buffer and in 40% EtOH/0.1 M HCI.
Fig. 13 compares oxycodone release from coated particle formulation 5 as a function of time in pH 6.8 phosphate buffer and in 40% EtOH/0.1 M HCI.
Fig. 14 compares oxycodone release from coated particle formulation 6 as a function of time in pH 6.8 phosphate buffer and in 40% EtOH/0.1 M HCI.
Fig. 15 compares oxycodone release from coated particle formulation 7 as a function of time in pH 6.8 phosphate buffer and in 40% EtOH/0.1 M HCI.
Fig. 16 compares oxycodone release from coated particle formulation 8 as a function of time in pH 6.8 phosphate buffer and in 40% EtOH/0.1 M HCI.
Fig. 17 compares oxycodone release from coated particle formulation 9 as a function of time in pH 6.8 phosphate buffer and in 40% EtOH/0.1 M HCI.
Fig. 18 compares oxycodone release from coated particle formulation 10 as a function of time in pH 6.8 phosphate buffer and in 40% EtOH/0.1 M HCI.
Fig. 19 compares oxycodone release from coated particle formulation 11 as a function of time in pH 6.8 phosphate buffer and in 40% EtOH/0.1 M HCI.

### Example 1 - Manufacture of particle formulations

### Formulations 1-2

Tetraethoxyorthosilane (TEOS) (3.50 kg, 16.80 moles) and hydrochloric acid (0.1 M solution in DI water, 840 mL, 83.95 mmoles) are added to a 10 L polypropylene beaker and stirred vigorously on a stirrer/hotplate. Oxycodone hydrochloride (252 g, 717.80 mmoles) is dissolved in deionised water (1579 mL) and then added to the TEOS/0.1 M HCI mixture. The resulting biphasic mixture is covered and left to stir at room temperature (NB. the reaction is exothermic in the initial stages and the temperature of the solution rises to ∼60-65 °C before naturally cooling to room temperature). The reaction gels after -72 hours, at which point the gel is transferred to HDPE trays, spread out and dried in a venting oven at 60 °C for 48 hours.

The resulting solid sol-gel glass chunks are reduced in size using a FitzMill Comminutor to give -500 µm sized particles of the drug-loaded sol-gel carrier. This material is then placed back in the oven and left to dry. After 40 hours the powder is removed from the oven and hand-milled using a mortar and pestle and subsequently sieved through 125 and 250 µm test sieves to give the desired particle size distribution. The hand-milled powder is then returned to the oven to dry for a further 20 hours.

The coating procedure is carried out using a Glatt GPCG 3 fluid bed-coater with a Würster insert attachment. A 40% (m/v) sodium phosphate coating solution (with a weight ratio of 1.89:1 P₂O₅:Na₂O) is first diluted to 10% (m/v). 400 g of 125-250 µm oxycodone-loaded sol-gel powder is added to the chamber of the coating machine, to the outer zone around the Würster cylinder. Fluidisation and heating is applied to the core powder for 10-15 minutes, with the following settings:
Air flow: 40 cfm
Inlet air temperature: 65 °C
Product temperature: 45 °C
Exhaust air temperature: 45 °C
Würster cylinder height: 10

The coating solution is then transferred to the coating chamber through a spray nozzle, via rubber tubing, using a peristaltic pump set at 3 rpm. The following settings are applied:
Air flow: 40 cfm
Inlet air temperature: 60 °C
Humidity dewpoint: 8.0 °C
Atomising pressure (to break coating solution into droplets): 1.2 bar
Filter shake time: 6 s
Filter shake repeat: 30 s
Product temperature: 37 °C
Exhaust air temperature: 37 °C

The amount of coating applied to the powder is calculated by measuring the weight loss of the coating solution. Usually, a target coating weight is between 1 and 5 % by weight of solid glass as a function of the original sol-gel powder.

**Table 1 - Quantity of coating applied to formulations 1-2**

| **Formulation** | **Weight % glass coating** |
|---|---|
| 1 | 5 |
| 2 | 10 |

### Formulations 3-11

Tetraethoxyorthosilane (TEOS) (3.50 kg, 16.80 moles) was weighed out into a 10 L polypropylene beaker. Oxycodone hydrochloride (252 g, 717.80 mmoles) is dissolved in 'extra' deionised water (see Table 2) using an overhead stirrer and then added to the TEOS. 0.1 M hydrochloric acid (see Table 2) is then added to the reaction flask and the resulting biphasic mixture stirred vigorously using a magnetic stirrer. A homogeneous sol is formed after approximately 25 minutes, and due to the exothermic nature of TEOS hydrolysis, coincides with a maximum temperature reached of -62 °C. The reaction beaker is covered and left to stir. After 18 hours the beaker covering is removed and the reaction left to stir at room temperature. Gellation occurs after a further X hours (see Table 3). The contents of the beaker are transferred to polypropylene drying trays and place in a vented oven at 60 °C to remove all residual solvents for Y hours (see Table 3). The resulting sol-gel glass chunks are then milled using a Retsch DM200 disc mill using progressively narrower gap widths (for individual protocols see Table 4). The milled powders are then sieved to the necessary particle size ranges (see Table 2) using 53, 90, 125, 180 and 250 µm test sieves and put in the oven for further drying for Z hours (see Table 3).

**Table 2 - Preparation variables for preparation of formulations 3-11**

| **Formulation** | **H⁺ eqs.** | **'Extra' DI water (mL)** | **0.1 M HCl (mL)** | **Particle size range (µm)** |
|---|---|---|---|---|
| 3 | 0.005 | 1579 | 840 | 125-250 |
| 4 | 0.01 | 739 | 1680 | 125-250 |
| 5 | 0.005 | 1579 | 840 | 125-250 |
| 6 | 0.0075 | 1159 | 1260 | 90-180 |
| 7 | 0.01 | 739 | 1680 | 53-125 |
| 8 | 0.01 | 739 | 1680 | 53-125 |
| 9 | 0.0075 | 1159 | 1260 | 90-180 |
| 10 | 0.005 | 1579 | 840 | 53-125 |
| 11 | 0.005 | 1579 | 840 | 53-125 |

**Table 3 - Drying schedules for preparation of formulations 3-11**

| **Formulation** | **X** **RT drying to gel point (hours)** | **Y** **Oven drying at 60 °C (hours)** | **Z** **Post-milling drying at 60 °C (hours)** |
|---|---|---|---|
| 3 | 70 | 52 | 18 |
| 4 | 97 | 50 | 52 |
| 5 | 70 | 69 | 23 |
| 6 | 76 | 53 | 22 |
| 7 | 71 | 52 | 24 |
| 8 | 71 | 57 | 28 |
| 9 | 46 | 95 | 17 |
| 10 | 25 | 71 | 15 |
| 11 | 25 | 90 | 16 |

**Table 4 - Milling protocols for preparation of formulations 3-11**

| **Formulation** | **Milling protocol:** Gap width µm (number of passes) |
|---|---|
| 3 | 508 (1), 254 (1), 203 (1), 254 (2) |
| 4 | 1003 (1), 508 (1), 254 (1), 229 (2), 203 (1) |
| 5 | 711 (1), 254 (3), 229 (1) |
| 6 | 711 (1), 305 (3), 229 (1), 178 (2) |
| 7 | 1003 (1), 305 (1), 229 (1), 178 (1), 127 (2), 102 (1) |
| 8 | 1003 (1), 508 (1), 254 (1), 178 (1), 127 (1), 102 (1) |
| 9 | 1003 (1), 508 (1), 254 (1), 203 (1), 178 (1), 152 (1), 127 (1) |
| 10 | 1003 (1), 508 (1), 254 (1), 178 (1), 127 (1), 102 (1) |
| 11 | 1003 (1), 508 (1), 254 (1), 178 (1), 127 (1), 102 (1) |

The coating procedure is carried out using a Glatt GPCG 3 fluid bed-coater with a Würster insert attachment. A 40% (m/v) sodium phosphate coating solution is first diluted to 10% (m/v). 450 g of the oxycodone-loaded sol-gel powder is added to the chamber of the coating machine, to the outer zone around the Würster cylinder. Fluidisation and heating is applied to the core powder for 10-15 minutes, with the following settings:
Air flow: 40 cfm
Inlet air temperature: 60-65 °C
Product temperature: 37 °C
Exhaust air temperature: 37 °C
Würster cylinder height: 10
The coating solution is then transferred to the coating chamber through a spray nozzle, via rubber tubing, using a peristaltic pump set at 2-5 rpm. The following settings are applied:
Air flow: 25-60 cfm
Inlet air temperature: 60-65 °C
Humidity dewpoint: 8-11 °C
Atomising pressure (to break coating solution into droplets): 1.2 bar
Filter shake time: 3/6 s
Filter shake repeat: 15/30 s
Product temperature: 37 °C
Exhaust air temperature: 37 °C
Würster cylinder height: 0-10

The amount of coating applied to the powder is calculated by measuring the weight loss of the coating solution. Usually, a target coating weight is between 1 and 5 % by weight of solid glass as a function of the original sol-gel powder (see Table 5).

**Table 5 - Quantity of coating applied to formulations 3-11**

| **Formulation** | **Weight % glass coating** |
|---|---|
| 3 | 1 |
| 4 | 4 |
| 5 | 5 |
| 6 | 3 |
| 7 | 1 |
| 8 | 5 |
| 9 | 3 |
| 10 | 1 |
| 11 | 5 |

### Example 2 - Dissolution studies

Aqueous solutions of the sodium phosphate and potassium silicate water-soluble glasses are first freeze-dried for 24 hours. The resulting solids are then milled and sieved to obtain glass powders of the size range 38-250 µm.

500 mg of the glass powder is added to 50 mL of the dissolution medium (pH 6.8 phosphate buffer or 40% EtOH/0.1 M HCI) and stirred. At the following time points - 5, 10, 20 and 30 minutes, a 10 mL aliquot is removed and filtered through a 0.45 µm PVDF syringe filter and analysed by ICP-OES analysis for sodium, potassium, silicon and phosphorus content as required. Simultaneously, the remaining solution (∼40 mL) is filtered through a fluted filter paper. Residual solids collected on the filter paper, and left in the reaction beaker, are dried thoroughly in the oven (typically 50-80 °C overnight) and weighed.

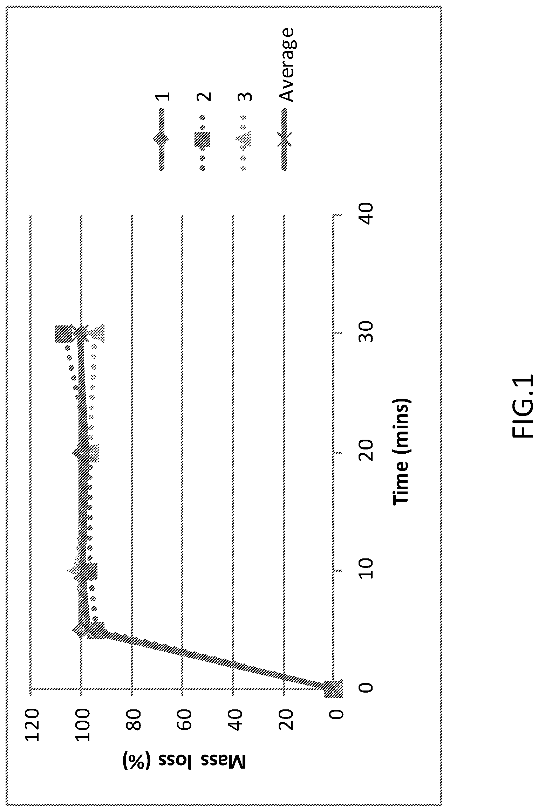

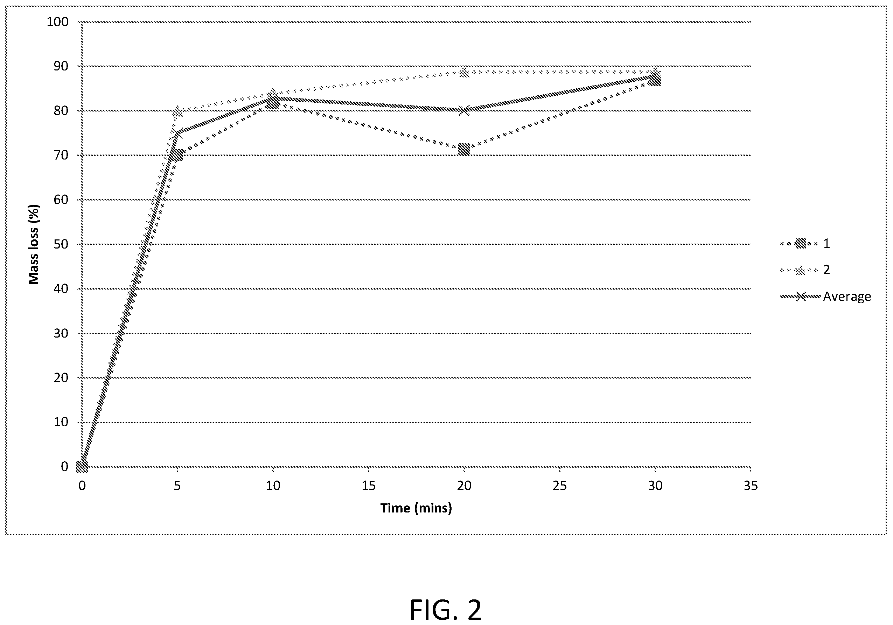

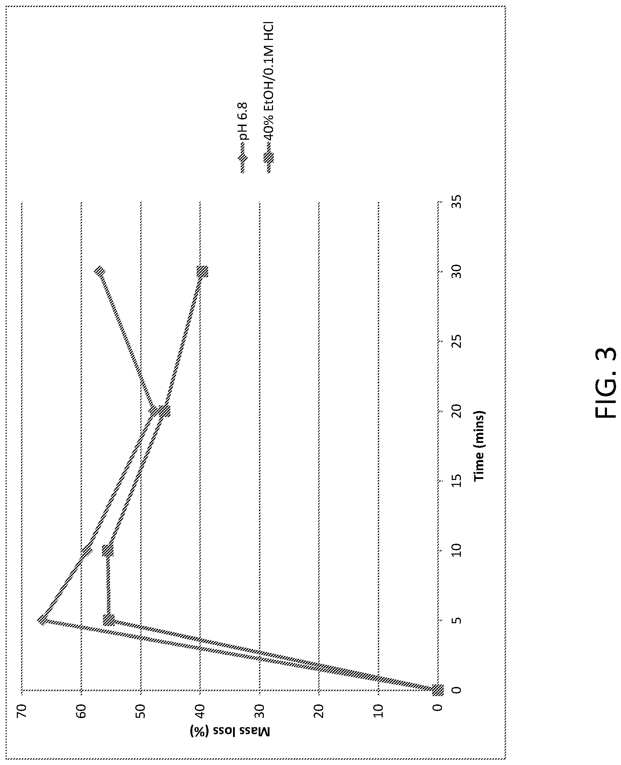

The results of the dissolution studies are provided in Figs. 1-4. The results show that dissolution behaviour from the phosphate glass is intrinsically different to the dissolution behaviour from the silicate glasses. Sodium phosphate and potassium silicate glasses both show fast dissolution rates within the first 5 minutes in both pH 6.8 phosphate buffer and 40% EtOH/0.1 M HCI media. The extent of dissolution for both glasses in pH 6.8 phosphate buffer is greater as compared with the extent of dissolution in 40% EtOH/0.1 M HCl.

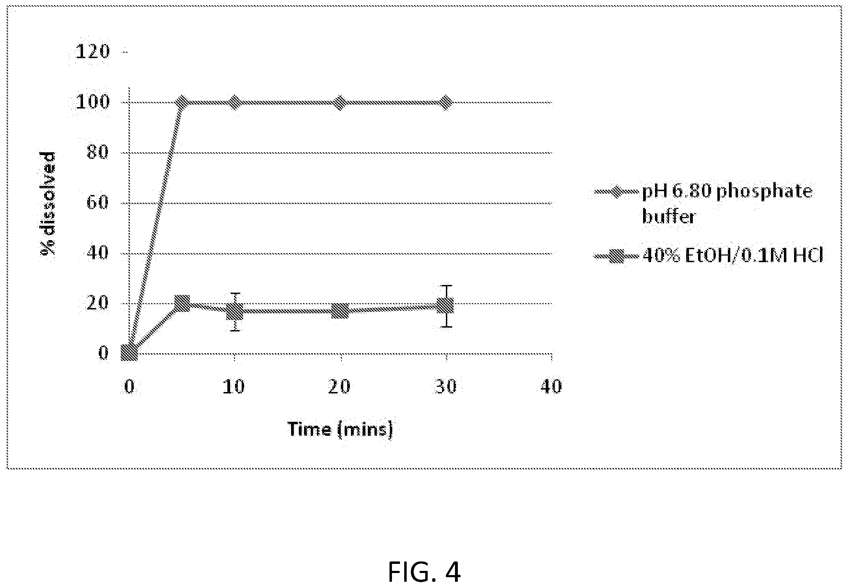

Using the procedure detailed in paragraph [0068] and [0069] above, a 1%(w/v) potassium silicate glass suspension with a ratio of 1.43:1 was made up and stirred in the dissolution medium (pH 6.8 phosphate buffer or 40% EtOH/0.1 M HCl) for 30 minutes. Aliquots of the suspension were taken at 5, 10, 20 and 30 minutes. The suspensions from the aliquots were then filtered and the resulting solids were dried at 80-95°C overnight before being weighed to determine the % weight loss (or % dissolution). Figure 4 illustrates a greater dissolution of the glass particles in the aqueous medium when compared to the organic medium after 5, 10, 20 and 30 minutes. Again, the majority of the dissolution is demonstrated as occurring within the first 5 minutes.

### Example 3 - Release studies

Standard conditions as specified by the United States Pharmacopoiea (USP) guidelines were followed, using a Distek USP I apparatus (basket dissolution tester).

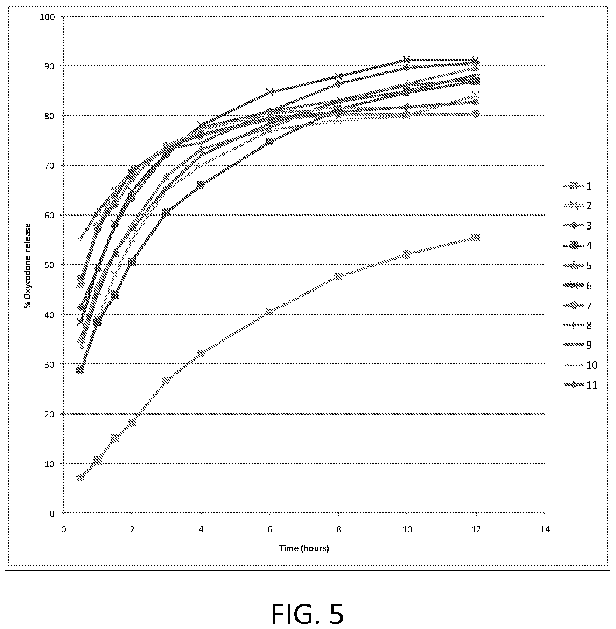

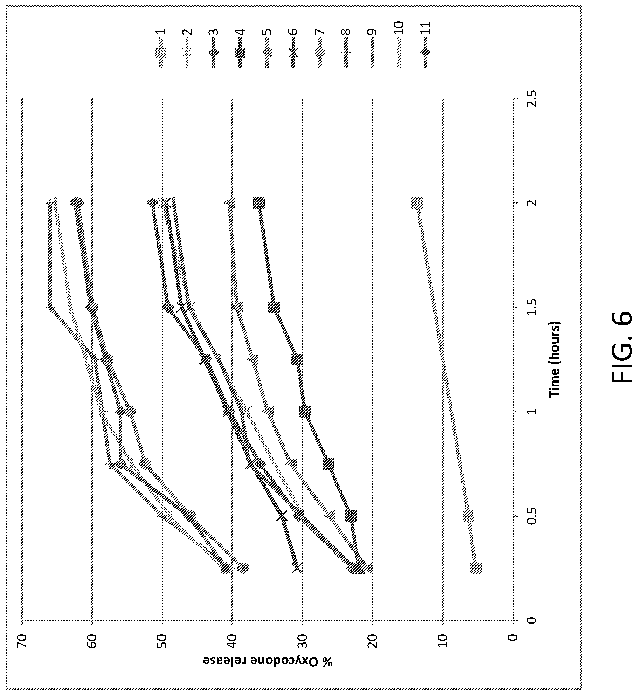

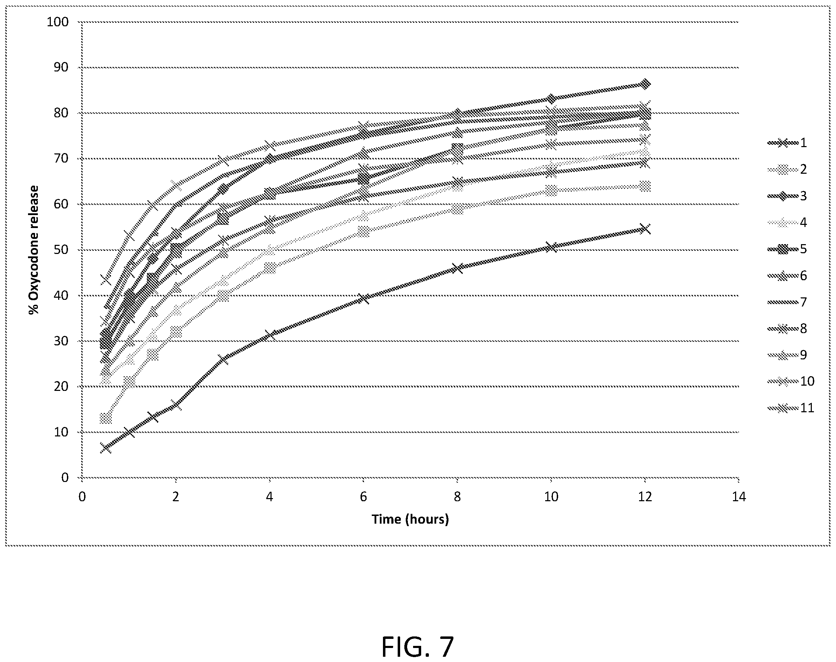

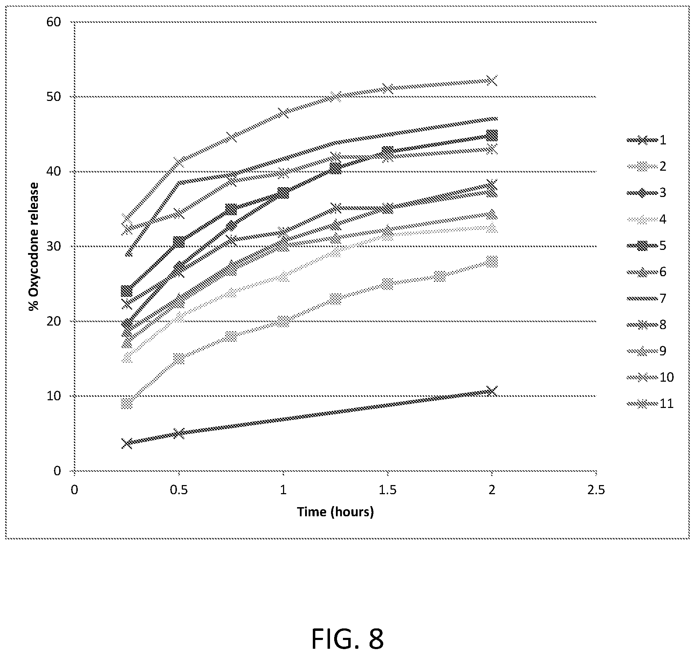

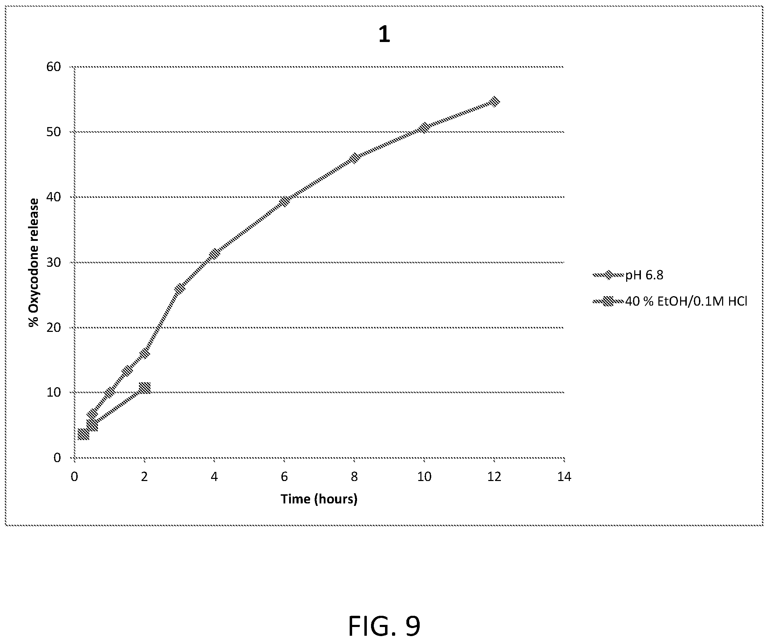

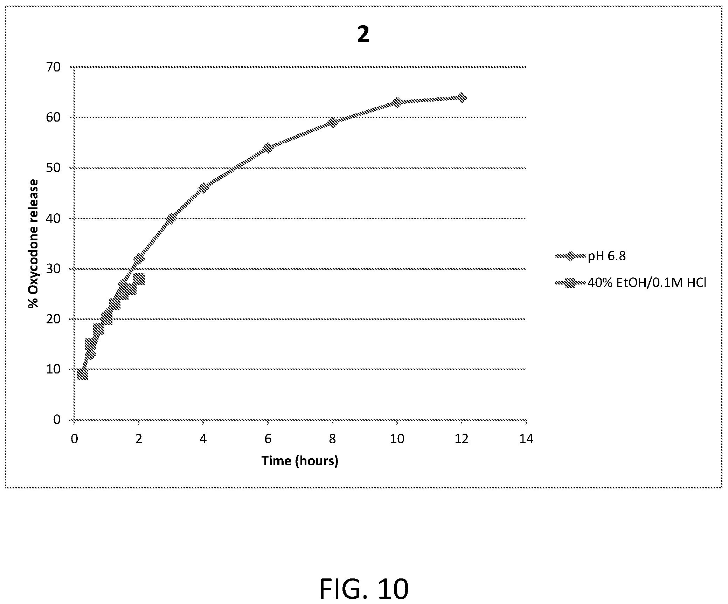

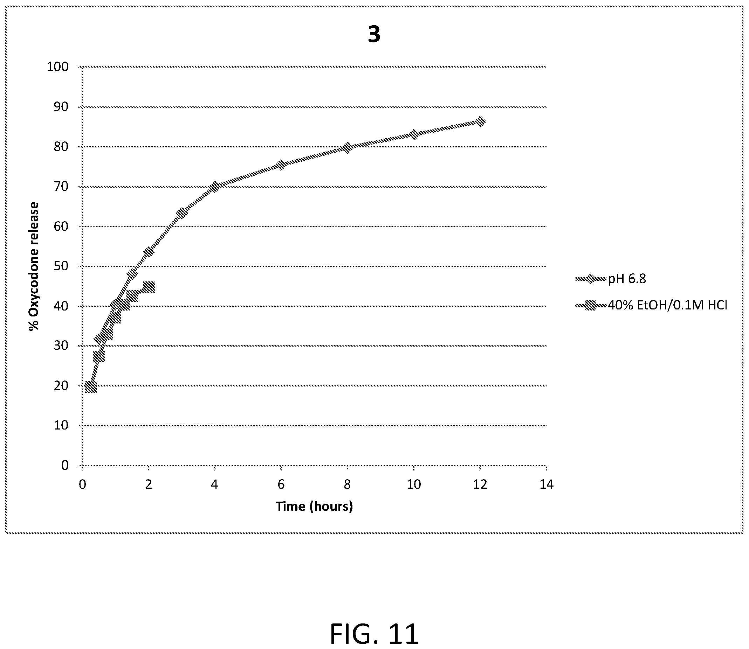

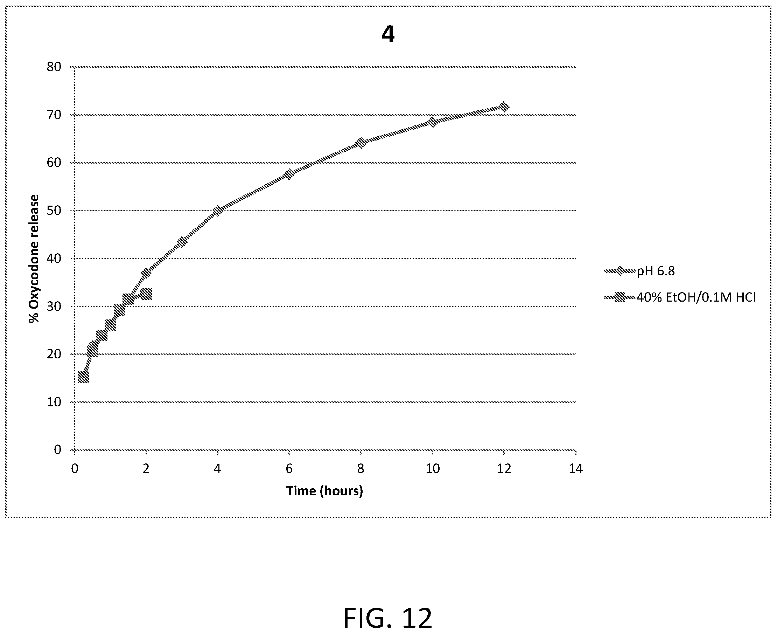

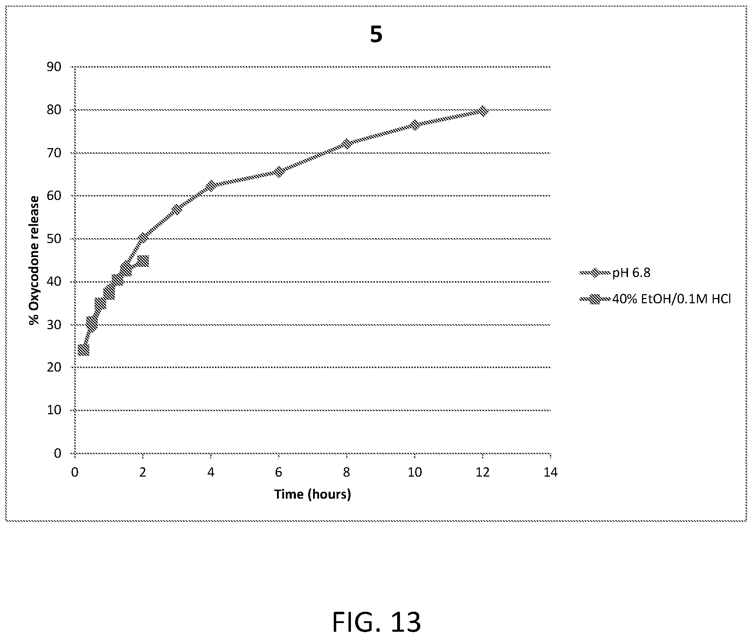

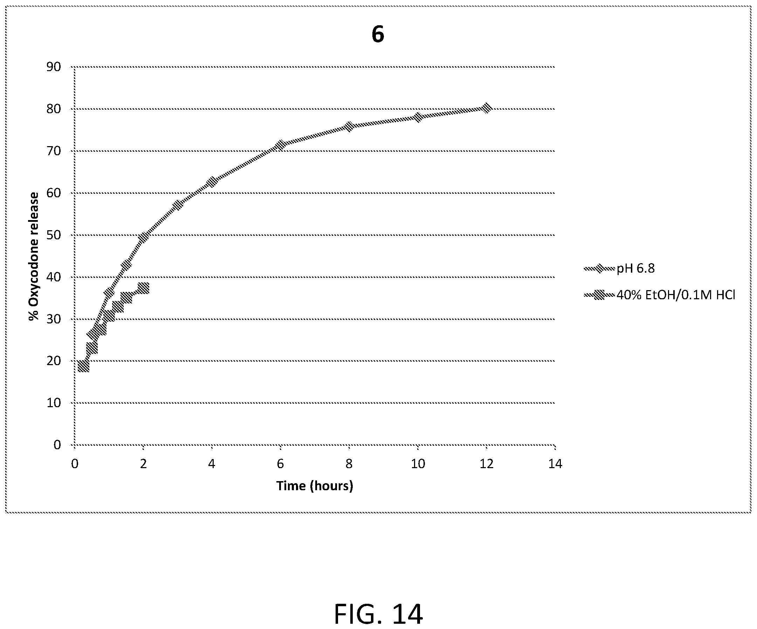

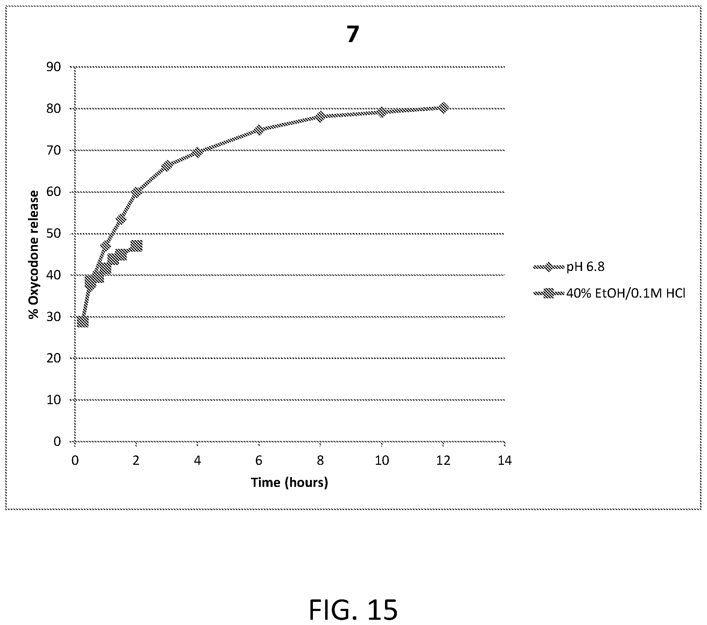

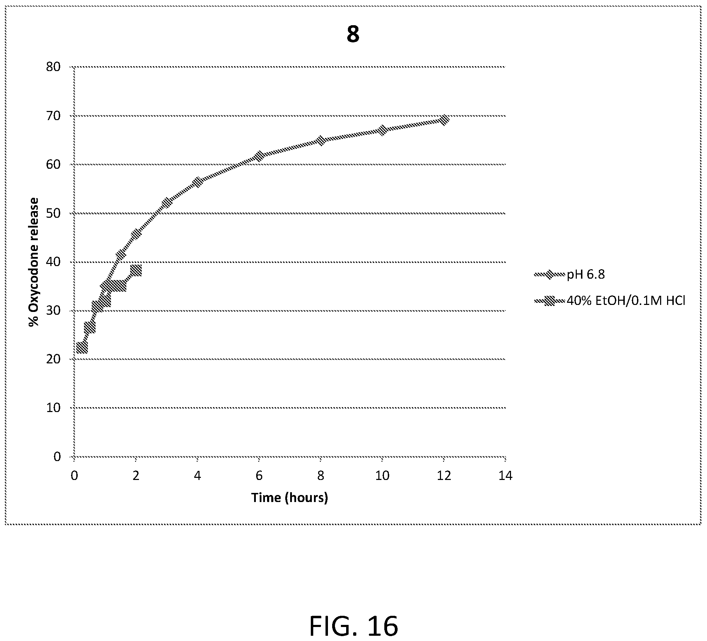

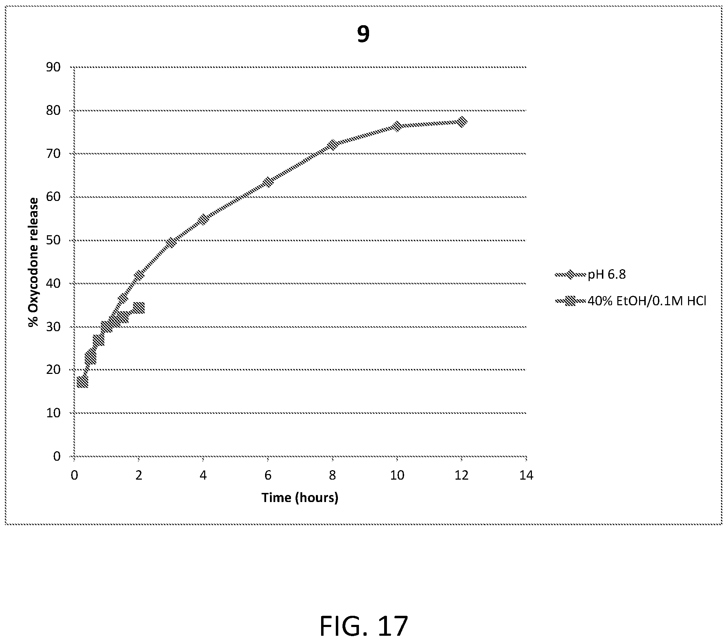

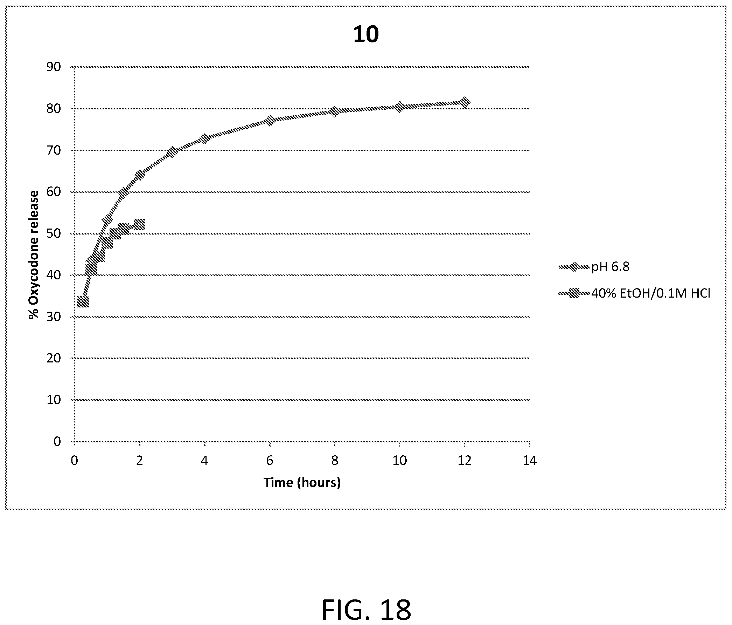

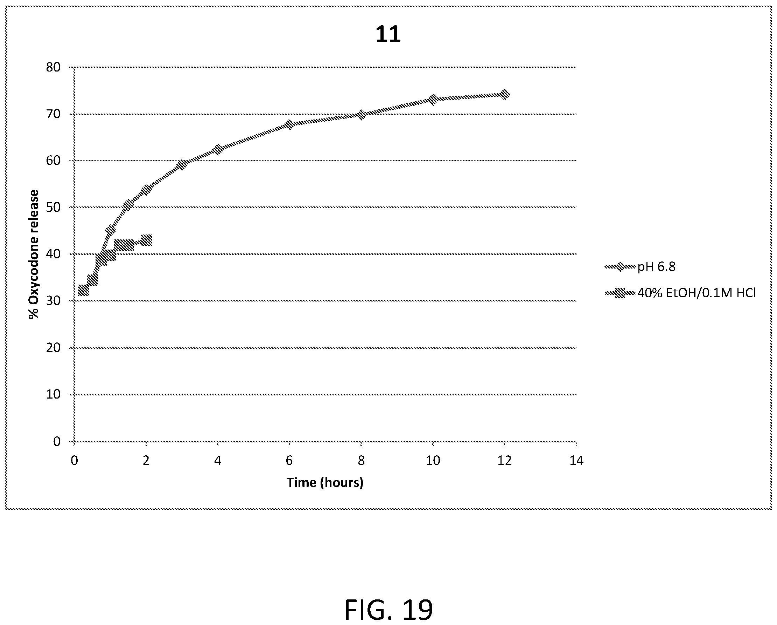

900 mL of dissolution media (pH 6.8 phosphate buffer and 40% EtOH/0.1 M HCI) was first de-gassed and then equilibrated to 37 °C ±0.5 °C. 200 mg of the formulation with the desired particle size distribution was added to 150 mesh baskets and submerged into the dissolution media and stirred at 100 rpm. At the necessary time points (every 15 minutes up to 2 hours for EtOH/HCI media and 0.5, 1, 1.5, 2, 3, 4, 6, 8, 10 and 12 hours for phosphate buffer) 9 mL of dissolution media was removed using a Distek autosampler and analysed for oxycodone content by UV/Vis spectroscopy. The results are provided in Tables 6 - 9 below, and in Figs. 5 - 19.

**Table 6 - Percentage oxycodone release in pH 6.8 phosphate buffer for uncoated particles of varying particle size correlated with increasing time**

| **Formulation** | **Time (hours)** | | | | | | | | | |
|---|---|---|---|---|---|---|---|---|---|---|
| | **0.5** | **1** | **1.5** | **2** | **3** | **4** | **6** | **8** | **10** | **12** |
| 1 | 7 | 11 | 15 | 18 | 27 | 32 | 41 | 48 | 52 | 56 |
| 2 | 29 | 39 | 48 | 55 | 65 | 70 | 77 | 79 | 80 | 84 |
| 3 | 42 | 49 | 58 | 63 | 72 | 78 | 81 | 86 | 90 | 91 |
| 4 | 29 | 38 | 44 | 51 | 60 | 66 | 75 | 81 | 85 | 87 |
| 5 | 35 | 46 | 52 | 58 | 68 | 73 | 78 | 83 | 86 | 90 |
| 6 | 38 | 49 | 58 | 65 | 73 | 78 | 85 | 88 | 91 | 91 |
| 7 | 47 | 58 | 62 | 67 | 74 | 76 | 79 | 80 | 80 | 80 |
| 8 | 55 | 61 | 65 | 69 | 73 | 74 | 81 | 83 | 86 | 87 |
| 9 | 33 | 44 | 53 | 57 | 66 | 72 | 78 | 83 | 85 | 88 |
| 10 | 46 | 58 | 65 | 68 | 74 | 77 | 80 | 82 | 82 | 83 |
| 11 | 46 | 57 | 63 | 69 | 73 | 76 | 80 | 81 | 82 | 83 |

**Table 7 - Percentage oxycodone release in 40% EtOH/0.1 M HCI for uncoated particles of varying particle size correlated with increasing time**

| **Formulation** | **Time (hours)** | | | | | | |
|---|---|---|---|---|---|---|---|
| | **0.25** | **0.5** | **0.75** | **1** | **1.25** | **1.5** | **2** |
| 1 | 5 | 6 | | | | | 14 |
| 2 | | 30 | | 38 | | 46 | 50 |
| 3 | 23 | 31 | 36 | 40 | 44 | 49 | 51 |
| 4 | 22 | 23 | 26 | 30 | 31 | 34 | 36 |
| 5 | 21 | 26 | 32 | 35 | 37 | 39 | 40 |
| 6 | 31 | 33 | 37 | 41 | 44 | 47 | 49 |
| 7 | 39 | 46 | 52 | 55 | 58 | 60 | 62 |
| 8 | 40 | 50 | 57 | 59 | 60 | 66 | 66 |
| 9 | 23 | 30 | 38 | 39 | 42 | 46 | 48 |
| 10 | 41 | 49 | 54 | 59 | 61 | 63 | 65 |
| 11 | 41 | 46 | 56 | 56 | 58 | 60 | 62 |

**Table 8 - Percentage oxycodone release in pH 6.8 phosphate buffer from coated particles of varying percentage coating levels correlated with increasing time**

| **Formulation and coating (C) content (wt%)** | **Time (hours)** | | | | | | | | | |
|---|---|---|---|---|---|---|---|---|---|---|
| | **0.5** | **1** | **1.5** | **2** | **3** | **4** | **6** | **8** | **10** | **12** |
| 1-C5 | 7 | 10 | 13 | 16 | 26 | 31 | 39 | 46 | 51 | 55 |
| 2-C10 | 13 | 21 | 27 | 32 | 40 | 46 | 54 | 59 | 63 | 64 |
| 3-C1 | 32 | 40 | 48 | 54 | 63 | 70 | 75 | 80 | 83 | 86 |
| 4-C4 | 22 | 26 | 32 | 37 | 43 | 50 | 58 | 64 | 68 | 72 |
| 5-C5 | 30 | 38 | 44 | 50 | 57 | 62 | 66 | 72 | 77 | 80 |
| 6-C3 | 26 | 36 | 43 | 49 | 57 | 63 | 71 | 76 | 78 | 80 |
| 7-C1 | 37 | 47 | 53 | 60 | 66 | 70 | 75 | 78 | 79 | 80 |
| 8-C2.5 | 27 | 35 | 41 | 46 | 52 | 56 | 62 | 65 | 67 | 69 |
| 9-C3 | 24 | 30 | 37 | 42 | 49 | 55 | 63 | 72 | 76 | 77 |
| 10-C1 | 43 | 53 | 60 | 64 | 70 | 73 | 77 | 79 | 80 | 82 |
| 11-C5 | 34 | 45 | 51 | 54 | 59 | 62 | 68 | 70 | 73 | 74 |

**Table 9 - Percentage oxycodone release in 40% EtOH/0.1 M HCI from coated particles of varying percentage coating levels correlated with increasing time**

| **Formulation and coating (C) content (wt%)** | **Time (hours)** | | | | | | |
|---|---|---|---|---|---|---|---|
| | **0.25** | **0.5** | **0.75** | **1** | **1.25** | **1.5** | **2** |
| 1-C5 | 4 | 5 | | | | | 11 |
| 2-C10 | 9 | 15 | 18 | 20 | 23 | 25 | 28 |
| 3-C1 | 20 | 27 | 33 | 37 | 40 | 43 | 45 |
| 4-C4 | 15 | 21 | 24 | 26 | 29 | 32 | 33 |
| 5-C5 | 22 | 28 | 32 | 34 | 37 | 39 | 41 |
| 6-C3 | 19 | 23 | 27 | 31 | 33 | 35 | 37 |
| 7-C1 | 29 | 39 | 40 | 42 | 44 | 45 | 47 |
| 8-C2.5 | 22 | 27 | 31 | 32 | 35 | 35 | 38 |
| 9-C3 | 17 | 23 | 27 | 30 | 31 | 32 | 34 |
| 10-C1 | 34 | 41 | 45 | 48 | 50 | 51 | 52 |
| 11-C5 | 32 | 34 | 39 | 40 | 42 | 42 | 43 |

In general, the range of release data shown demonstrates that by varying the sol-gel processing parameters; amount of acid catalyst and particle size distribution, elution of the encapsulated active can be controlled both in terms of its rate and extent of release.

Once coated, the rate of release of the encapsulated drug from the core particles is reduced. Typically the rate of release is reduced to a greater extent when the dissolution medium is 40% EtOH/0.1 M HCI than when the dissolution medium is pH 6.8 phosphate buffer. The results also suggest that the reduction in release rate can be correlated with the amount of glass coating present.

### Example 4 - Particle size

Table 10 below provides particle size data for formulation 2 particles having a particle size of 125-250 µm. The data show that particle size is not substantially altered by the quantity of coating applied to the core substrate.

**Table 10 - Particle size data for formulation 2 125-250 um particles coated to a varying extent**

| **Quantity of coating** | **Particle size (µm)** | | |
|---|---|---|---|
| | **d (0.1)** | **d (0.5)** | **d (0.9)** |
| Uncoated | 121.8 | 213.4 | 354.6 |
| 1 wt% | 107.8 | 204.7 | 354.3 |
| 2 wt% | 113.6 | 208.5 | 349.3 |
| 5 wt% | 114.1 | 214.1 | 368.6 |
| 10 wt% | 105.5 | 205.0 | 354.1 |

Table 11 below shows that for the disc milling process employed, the size distributions for the products formed are consistent within the nominal size ranges desired.

**Table 11 - Particle size data for uncoated particles of varying size ranges and distributions**

| **Formulation** | **Particle size (µm)** | | | |
|---|---|---|---|---|
| | **d (0.1)** | **d (0.5)** | **d (0.9)** | **Nominal size range** |
| 1 | 136.1 | 239.0 | 405.5 | 125-250 |
| 2 | 121.8 | 213.4 | 354.6 | 125-250 |
| 3 | 93.7 | 198.7 | 346.1 | 125-250 |
| 4 | 91.0 | 197.7 | 352.2 | 125-250 |
| 5 | 98.9 | 211.9 | 423.2 | 125-250 |
| 6 | 56.7 | 136.6 | 248.4 | 90-180 |
| 7 | 23.9 | 84.0 | 184.9 | 53-125 |
| 8 | 28.1 | 81.8 | 166.7 | 53-125 |
| 9 | 51.9 | 132.8 | 244.5 | 90-180 |
| 10 | 25.1 | 81.4 | 174.5 | 53-125 |
| 11 | 26.0 | 79.6 | 160.5 | 53-125 |

### REFERENCES

1. National Institute on Drug Abuse (NIDA) NIDA Community Drug Alert Bulletin: Prescription Drugs. Bethesda, Md: U.S. Department of Health and Human Services; 2005. NIH Pub. No. 05-0580. Available at: http://archives.drugabuse.gov/prescripalert/. Accessed November 1, 2011.
2. Passik SD, Kirsh KL, Donaghy KB, Portenoy RK. Pain and aberrant drug-related behaviors in medically ill patients with and without histories of substance abuse. Clin J Pain. 2006;22(2):173-181.[PubMed]

## Claims

1. A particle comprising:
a core substrate having a plurality of pores, and
a coating substantially encapsulating the core substrate,
the core substrate comprising at least one active ingredient present within the pores, and the coating being formed from an alkali phosphate glass or an alkali silicate glass, wherein the core substrate is selected from the group consisting of amorphous silica, glasses, clays, zeolites and ceramics and wherein the alkali phosphate or alkali silicate has a greater dissolution rate in aqueous media than in alcoholic media.

2. The particle of claim 1, wherein any one or more of the following statements applies:
i. the at least one active ingredient is a pharmaceutically active compound;
ii. the particle is a microparticle.

3. The particle of claim 1 or 2, wherein any one or more of the following statements applies:
i. the alkali phosphate and alkali silicate glasses comprise at least one oxide selected from alkali metal oxides and alkaline earth metal oxides;
ii. the alkali phosphate and alkali silicate glasses comprise Na₂O, and optionally one or more other oxides selected from calcium oxide and magnesium oxide;
iii. the alkali silicate glass has a weight ratio of silica to total alkali oxide of 4:1 to 1:1;
iv. the alkali phosphate glass comprises 30-50 mol% phosphate;
v. the alkali phosphate glass comprises 40-48 mol% phosphate;
vi. the phosphate is provided as P₂O₅;
vii. the coating is formed from an alkali phosphate glass consisting of 46 mol% P₂O₅ and 54 mol% Na₂O.

4. The particle of claim 1, 2 or 3, wherein any one or more of the following statements applies:
i. the core substrate is produced via a sol-gel technique;
ii. the core substrate is amorphous silica;
iii. the particle has a diameter of 50 - 350 µm;
iv. the particle has a diameter of 125 - 250 µm.

5. The particle of any preceding claim, wherein any one or more of the following statements applies:
i. the particle comprises 1 - 10 wt% of the coating;
ii. the particle comprises 1 - 5 wt% of the coating;
iii. the particle comprises 2 - 5 wt% of the coating.

6. The particle of any preceding claim, wherein any one or more of the following statements applies:
i. the plurality of pores each have a diameter of 1.5 - 50 nm;
ii. the plurality of pores each have a diameter of 1.5 - 30 nm;
iii. the core substrate has a pore volume of 1 x 10⁻³ - 10 cm³g⁻¹;
iv. the core substrate has a pore volume of 5 x 10⁻³ - 5 cm³g⁻¹.

7. The particle of any preceding claim, wherein any one or more of the following statements applies:
i. the at least one active ingredient comprises an opioid or an opioid derivative;
ii. the at least one active ingredient comprises oxycodone or a pharmaceutically acceptable salt thereof.

8. A process for the preparation of a plurality of particles as claimed in any preceding claim, the process comprising the steps of:
a) providing a plurality of core substrates each comprising a plurality of pores as claimed in any preceding claims, wherein the pores comprise at least one active ingredient as claimed in any preceding claim, and
b) contacting the plurality of core substrates with an alkali phosphate glass or an alkali silicate glass as claimed in any preceding claim, such that the plurality of core substrates become coated with the alkali phosphate glass or the alkali silicate glass.

9. The process of claim 8, wherein step b) comprises the step of fluidizing the plurality of core substrates in the presence of an alkali phosphate glass or an alkali silicate glass as claimed in any preceding claim, such that the plurality of core substrates become coated with the alkali phosphate glass or the alkali silicate glass.

10. The process of claim 8 or 9, wherein step a) comprises the steps of:
a1) providing a liquid mixture of core substrate precursors,
a2) subjecting the liquid mixture to conditions suitable to form a plurality of core substrates, and
a3) isolating the resulting core substrates comprising the at least one active ingredient,
and wherein the at least one active ingredient is introduced either before, during or after step a2).

11. The process of claim 10, wherein any one or more of the following statements applies:
i. step a1) comprises providing a liquid mixture of core substrate precursors in an aqueous solution;
ii. the at least one active ingredient is contacted with the liquid mixture of core substrate precursors prior to step a2);
iii. the at least one active ingredient is provided in aqueous solution;
iv. step a3) comprises drying the resulting core substrates, then milling the dried core substrates.

12. The process of any of claims 8 to 11, wherein the alkali phosphate glass or alkali silicate glass used in step b) is provided as an aqueous solution comprising 3-25% (m/v) of the glass,
optionally wherein either or both of the following statements applies:
i. step b) comprises introducing the solution of alkali phosphate glass or alkali silicate glass to a fluidized bed of core substrates, optionally wherein the solution of alkali phosphate glass or alkali silicate glass is introduced to the fluidized bed of core substrates at a pressure of 0.8-1.5 bar.
ii. step b) comprises introducing the solution of alkali phosphate glass or alkali silicate glass to a fluidized bed of core substrates and the solution of alkali phosphate glass or alkali silicate glass is introduced under sufficient pressure to provide a mist of glass, optionally wherein the solution of alkali phosphate glass or alkali silicate glass is introduced to the fluidized bed of core substrates at a pressure of 0.8-1.5 bar.

13. The process of any of claims 9 to 12, wherein any one or more of the following statements applies:
i. the exhaust air is maintained at a temperature of 30 - 50°C during fluidization;
ii. the exhaust air is maintained at a temperature of 35 - 47°C during fluidization;
iii. the core substrates are maintained at a temperature of 30 - 50°C during fluidization;
iv. the core substrates are maintained at a temperature of 35 - 47°C during fluidization.

14. Tamper proof particles comprising particles of claim 7.

15. A solid dosage form comprising particles of claim 7.

## Patentansprüche

1. Partikel, umfassend:
ein Kernsubstrat, das eine Vielzahl von Poren aufweist, und
eine Beschichtung, die im Wesentlichen das Kernsubstrat einkapselt, das Kernsubstrat umfassend mindestens einen Wirkstoff, der innerhalb der Poren vorhandenen ist, und wobei die Beschichtung aus einem Alkaliphosphatglas oder einem Alkalisilikatglas gebildet ist, wobei das Kernsubstrat ausgewählt ist aus der Gruppe, bestehend aus amorphem Siliciumdioxid, Gläsern, Tonen, Zeolithen und Keramiken, und wobei das Alkaliphosphat oder Alkalisilikat eine größere Auflösungsrate in wässrigen Medien als in alkoholischen Medien aufweist.

2. Partikel nach Anspruch 1, wobei eine oder mehrere der folgenden Aussagen zutreffen:
i. der mindestens eine Wirkstoff ist eine pharmazeutisch wirksame Verbindung;
ii. das Partikel ist ein Mikropartikel.

3. Partikel nach Anspruch 1 oder 2, wobei eine oder mehrere der folgenden Aussagen zutreffen:
i. die Alkaliphosphat- und Alkalisilikatgläser umfassen mindestens ein Oxid, das ausgewählt ist aus Alkalimetalloxiden und Erdalkalimetalloxiden;
ii. die Alkaliphosphat- und Alkalisilikatgläser umfassen Na₂O und optional ein oder mehrere andere Oxide, ausgewählt aus Calciumoxid und Magnesiumoxid;
iii. das Alkalisilikatglas weist ein Gewichtsverhältnis von Siliciumdioxid zu Gesamtalkalioxid von 4:1 bis 1:1 auf;
iv. das Alkaliphosphatglas umfasst 30-50 Mol-% Phosphat;
v. das Alkaliphosphatglas umfasst 40-48 Mol-% Phosphat;
vi. das Phosphat ist als P₂O₅ bereitgestellt;
vii. die Beschichtung ist aus einem Alkaliphosphatglas gebildet, das aus 46 Mol% P₂O₅ und 54 Mol-% Na₂O besteht.

4. Partikel nach Anspruch 1, 2 oder 3, wobei eine oder mehrere der folgenden Aussagen zutreffen:
i. das Kernsubstrat wird mittels eines Sol-Gel-Verfahrens hergestellt;
ii. das Kernsubstrat ist amorphes Siliziumdioxid;
iii. das Partikel weist einen Durchmesser von 50 - 350 µm auf;
iv. das Partikel weist einen Durchmesser von 125 - 250 µm auf.

5. Partikel nach einem der vorherigen Ansprüche, wobei eine oder mehrere der folgenden Aussagen zutreffen:
i. das Partikel umfasst 1-10 Gewichtsprozent der Beschichtung;
ii. das Partikel umfasst 1-5 Gewichtsprozent der Beschichtung;
iii. das Partikel umfasst 2-5 Gewichtsprozent der Beschichtung.

6. Partikel nach einem der vorherigen Ansprüche, wobei eine oder mehrere der folgenden Aussagen zutreffen:
i. die Vielzahl von Poren weisen jeweils einen Durchmesser von 1,5-50 nm auf;
ii. die Vielzahl von Poren weisen jeweils einen Durchmesser von 1,5-30 nm auf;
iii. das Kernsubstrat weist ein Porenvolumen von 1 x 10⁻³ - 10 cm³g⁻¹ auf;
iv. das Kernsubstrat weist ein Porenvolumen von 5 x 10⁻³ - 5 cm³g⁻¹ auf.

7. Partikel nach einem der vorherigen Ansprüche, wobei eine oder mehrere der folgenden Aussagen zutreffen:
i. der mindestens eine Wirkstoff umfasst ein Opioid oder ein Opioidderivat;
ii. der mindestens eine Wirkstoff umfasst Oxycodon oder ein pharmazeutisch annehmbares Salz davon.

8. Verfahren zur Herstellung einer Vielzahl von Partikeln nach einem der vorherigen Ansprüche, das Verfahren umfassend die folgenden Schritte:
a) Bereitstellen einer Vielzahl von Kernsubstraten, jeweils umfassend eine Vielzahl von Poren nach einem der vorherigen Ansprüche, wobei die Poren mindestens einen Wirkstoff nach einem der vorherigen Ansprüche umfassen, und
b) Inkontaktbringen der Vielzahl von Kernsubstraten mit einem Alkaliphosphatglas oder einem Alkalisilikatglas nach einem der vorherigen Ansprüche, sodass die Vielzahl von Kernsubstraten mit dem Alkaliphosphatglas oder dem Alkalisilikatglas beschichtet wird.

9. Verfahren nach Anspruch 8, wobei Schritt b) den Schritt eines Fluidisierens der Vielzahl von Kernsubstraten in Anwesenheit eines Alkaliphosphatglases oder eines Alkalisilikatglases nach einem der vorherigen Ansprüche umfasst, sodass die Vielzahl von Kernsubstraten mit dem Alkaliphosphatglas oder dem Alkalisilikatglas beschichtet wird.

10. Verfahren nach Anspruch 8 oder 9, wobei Schritt a) die folgenden Schritte umfasst:
a1) Bereitstellen eines flüssigen Gemischs von Kernsubstratvorläufern,
a2) Aussetzen des flüssigen Gemischs an Bedingungen, die geeignet sind, eine Vielzahl von Kernsubstraten zu bilden, und
a3) Isolieren der resultierenden Kernsubstrate, umfassend den mindestens einen Wirkstoff,
und wobei der mindestens eine Wirkstoff entweder vor, während oder nach Schritt a2) eingebracht wird.

11. Verfahren nach Anspruch 10, wobei eine oder mehrere der folgenden Aussagen zutreffen:
i. Schritt a1) umfasst ein Bereitstellen eines flüssigen Gemischs von Kernsubstratvorläufern in einer wässrigen Lösung;
ii. der mindestens eine Wirkstoff wird vor Schritt a2) mit dem flüssigen Gemisch von Kernsubstratvorläufern in Kontakt gebracht;
iii. der mindestens eine Wirkstoff wird in wässriger Lösung bereitgestellt;
iv. Schritt a3) umfasst ein Trocknen der resultierenden Kernsubstrate und dann ein Mahlen der getrockneten Kernsubstrate.

12. Verfahren nach einem der Ansprüche 8 bis 11, wobei das in Schritt b) verwendete Alkaliphosphatglas oder Alkalisilikatglas als wässrige Lösung bereitgestellt wird, umfassend 3-25 % (Masse/Volumen) des Glases,
optional wobei eine der beiden oder beide der folgenden Aussagen zutreffen:
i. Schritt b) umfasst ein Einbringen der Lösung von Alkaliphosphatglas oder Alkalisilikatglas in ein Wirbelbett aus Kernsubstraten, optional wobei die Lösung von Alkaliphosphatglas oder Alkalisilikatglas bei einem Druck von 0,8-1,5 bar in das Wirbelbett aus Kernsubstraten eingebracht wird,
ii. Schritt b) umfasst ein Einbringen der Lösung von Alkaliphosphatglas oder Alkalisilikatglas in ein Wirbelbett aus Kernsubstraten, und die Lösung von Alkaliphosphatglas oder Alkalisilikatglas wird unter ausreichendem Druck eingebracht, um einen Glasnebel zu erzeugen, optional wobei die Lösung von Alkaliphosphatglas oder Alkalisilikatglas bei einem Druck von 0,8 bis 1,5 bar in das Wirbelbett aus Kernsubstraten eingebracht wird.

13. Verfahren nach einem der Ansprüche 9 bis 12, wobei eine oder mehrere der folgenden Aussagen zutreffen:
i. die Abluft wird während des Fluidisierens auf einer Temperatur von 30-50 °C gehalten;
ii. die Abluft wird während des Fluidisierens auf einer Temperatur von 35-47 °C gehalten;
iii. die Kernsubstrate werden während des Fluidisierens auf einer Temperatur von 30-50 °C gehalten;
iv. die Kernsubstrate werden während des Fluidisierens auf einer Temperatur von 35-47 °C gehalten.

14. Fälschungssichere Partikel, umfassend Partikel nach Anspruch 7.

15. Feste Darreichungsform, umfassend Partikel nach Anspruch 7.

## Revendications

1. Particule comprenant :
un substrat formant noyau ayant une pluralité de pores, et
un revêtement encapsulant substantiellement le substrat formant noyau,
le substrat formant noyau comprenant au moins un ingrédient actif présent dans les pores, et le revêtement étant formé à partir d'un verre de phosphate alcalin ou d'un verre de silicate alcalin, où le substrat formant noyau est sélectionné dans le groupe constitué de silice amorphe, verres, argiles, zéolites et céramiques, et où le phosphate alcalin ou le silicate alcalin a un taux de dissolution plus grand dans les milieux aqueux que dans les milieux alcooliques.

2. Particule selon la revendication 1, à laquelle s'appliquent une ou plusieurs des affirmations suivantes :
i. le ou les ingrédients actifs sont un composé ayant une activité pharmaceutique ;
ii. la particule est une microparticule.

3. Particule selon la revendication 1 ou 2, à laquelle s'appliquent une ou plusieurs des affirmations suivantes :
i. les verres de phosphates alcalins et de silicates alcalins comprennent au moins un oxyde sélectionné entre oxydes métalliques alcalins et oxydes de métaux alcalino-terreux ;
ii. les verres de phosphates alcalins et de silicates alcalins comprennent Na₂O, et optionnellement un ou plusieurs autres oxydes sélectionnés entre l'oxyde de calcium et l'oxyde de magnésium ;
iii. le verre de silicate alcalin a un rapport de poids de la silice à l'oxyde alcalin total de 4:1 à 1:1 ;
iv. le verre de phosphate alcalin comprend 30-50 mol % de phosphate ;
v. le verre de phosphate alcalin comprend 40-48 mol % de phosphate ;
vi. le phosphate est fourni sous la forme de P₂O₅ ;
vii. le revêtement est formé à partir d'un verre de phosphate alcalin constitué de 46 mol % P₂O₅ et 54 mol % Na₂O.

4. Particule selon la revendication 1, 2 ou 3, à laquelle s'appliquent une ou plusieurs des affirmations suivantes :
i. le substrat formant noyau est produit par une technique sol-gel ;
ii. le substrat formant noyau est en silice amorphe ;
iii. la particule a un diamètre de 50 - 350 µm ;
iv. la particule a un diamètre de 125 - 250 µm.

5. Particule selon l'une quelconque des revendications précédentes, à laquelle s'appliquent une ou plusieurs des affirmations suivantes :
i. la particule comprend 1-10 % en poids du revêtement ;
ii. la particule comprend 1-5 % en poids du revêtement ;
iii. la particule comprend 2-5 % en poids du revêtement.

6. Particule selon l'une quelconque des revendications précédentes, à laquelle s'appliquent une ou plusieurs des affirmations suivantes :
i. chacun de la pluralité de pores a un diamètre de 1,5 - 50 nm ;
ii. chacun de la pluralité de pores a un diamètre de 1,5 - 30 nm ;
iii. le substrat formant noyau a un volume de pores de 1 x 10⁻³ - 10 cm³g⁻¹ ;
iv. le substrat formant noyau a un volume de pores de 5 x 10⁻³ - 5 cm³g⁻¹.

7. Particule selon l'une quelconque des revendications précédentes, à laquelle s'appliquent une ou plusieurs des affirmations suivantes :
i. le ou les ingrédients actifs comprennent un opioïde ou un dérivé d'opioïde ;
ii. le ou les ingrédients actifs comprennent l'oxycodone ou un sel de celui-ci de qualité pharmaceutique.

8. Procédé pour la préparation d'une pluralité de particules selon l'une quelconque des revendications précédentes, le procédé comprenant les étapes de :
a) fourniture d'une pluralité de substrats formant noyaux comprenant chacun une pluralité de pores selon l'une quelconque des revendications précédentes, où les pores comprennent au moins un ingrédient actif selon l'une quelconque des revendications précédentes, et
b) mise en contact de la pluralité de substrats formant noyaux avec un verre de phosphate alcalin ou un verre de silicate alcalin selon l'une quelconque des revendications précédentes, de telle sorte que la pluralité de substrats formant noyaux soient enduits du verre de phosphate alcalin ou du verre de silicate alcalin.

9. Procédé selon la revendication 8, dans lequel l'étape b) comprend l'étape de fluidisation de la pluralité de substrats formant noyaux en présence d'un verre de phosphate alcalin ou d'un verre de silicate alcalin selon l'une quelconque des revendications précédentes, de telle sorte que la pluralité de substrats formant noyaux soient enduits du verre de phosphate alcalin ou du verre de silicate alcalin.

10. Procédé selon la revendication 8 ou 9, dans lequel l'étape a) comprend les étapes de :
a1) fourniture d'un mélange liquide de précurseurs des substrats formant noyaux,
a2) exposition du mélange liquide à des conditions convenant à la formation d'une pluralité de substrats formant noyaux, et
a3) isolation des substrats formant noyaux résultants comprenant le ou les ingrédients actifs,
et dans lequel le ou les ingrédients actifs sont introduit soit avant, soit pendant, soit après l'étape a2).

11. Procédé selon la revendication 10, auquel s'appliquent une ou plusieurs des affirmations suivantes :
i. l'étape a1) comprend la fourniture d'un mélange liquide de précurseurs des substrats formant noyaux dans une solution aqueuse ;
ii. le ou les ingrédients actifs sont mis en contact avec le mélange liquide de précurseurs des substrats formant noyaux avant l'étape a2) ;
iii. le ou les ingrédients actifs sont fournis dans une solution aqueuse ;
iv. l'étape a3) comprend le séchage des substrats formant noyaux résultants, puis le broyage des substrats formant noyaux séchés.

12. Procédé selon l'une quelconque des revendications 8 à 11, dans lequel le verre de phosphate alcalin ou le verre de silicate alcalin utilisé dans l'étape b) est fourni sous forme de solution aqueuse comprenant 3-25 % (masse/volume) du verre,
optionnellement auquel s'appliquent l'une ou les deux des affirmations suivantes :
i. l'étape b) comprend l'introduction de la solution de verre de phosphate alcalin ou de verre de silicate alcalin dans un lit fluidisé de substrats formant noyaux, optionnellement où la solution de verre de phosphate alcalin ou de verre de silicate alcalin est introduite dans le lit fluidisé de substrats formant noyaux à une pression de 0,8-1,5 bar.
ii. l'étape b) comprend l'introduction de la solution de verre de phosphate alcalin ou de verre de silicate alcalin dans un lit fluidisé de substrats formant noyaux et la solution de verre de phosphate alcalin ou de verre de silicate alcalin est introduite à une pression suffisante pour fournir un brouillard de verre, optionnellement où la solution de verre de phosphate alcalin ou de verre de silicate alcalin est introduite dans le lit fluidisé de substrats formant noyaux à une pression de 0,8-1,5 bar.

13. Procédé selon l'une quelconque des revendications 9 à 12, auquel s'appliquent une ou plusieurs des affirmations suivantes :
i. l'air d'évacuation est maintenu à une température de 30 - 50°C pendant la fluidisation ;
ii. l'air d'évacuation est maintenu à une température de 35 - 47°C pendant la fluidisation ;
iii. les substrats formant noyaux sont maintenus à une température de 30 - 50°C pendant la fluidisation ;
iv. les substrats formant noyaux sont maintenus à une température de 35 - 47°C pendant la fluidisation.

14. Particules inviolables comprenant les particules de la revendication 7.

15. Forme pharmaceutique solide comprenant les particules de la revendication 7.
